# FASCICULE DE BREVET EUROPEEN

(11) **EP 3 548 052 B1**
(45) Date de publication et mention de la délivrance du brevet: **16.06.2021**
(21) Numéro de dépôt: 17816937.1
(22) Date de dépôt: 30.11.2017
(51) Int. Cl.: A61K 35/741, A61K 45/06, A61K 35/744, A61P 33/00, A61P 31/04, A01N 63/20, A01N 63/22, A61K 9/00, A61K 9/12

(54) **UTILISATION D'UNE COMPOSITION BACTÉRIENNE POUR TRAITER DES INFECTIONS DU PIED CHEZ LES ONGULÉS**
VERWENDUNG EINER BAKTERIELLEN ZUSAMMENSETZUNG ZUR BEHANDLUNG VON FUSSINFEKTIONEN BEI HUFTIEREN
USE OF A BACTERIAL COMPOSITION FOR TREATING FOOT INFECTIONS OF UNGULATES

(30) Priorité: 30.11.2016 FR 1661749
(43) Date de publication de la demande: 09.10.2019
(73) Titulaire: Nolivade, 53810 Changé (FR)
(72) Inventeur: LAYUS, Michel, 22560 Pleumeur Bodou (FR); BRAME, Alexandre, 35000 Rennes (FR)
(74) Mandataire: LLR
(86) Numéro de dépôt international: PCT/FR2017/053290
(87) Numéro de publication internationale: WO 2018/100292

(56) Documents cités:
- WO-A1-98/47374
- WO-A1-2013/178947
- WO-A1-2014/151837
- FR-A1- 3 026 643

## Description

L'invention concerne l'utilisation d'une composition pour traiter les pathologies animales.

Dans les élevages bovins, caprins et ovins modernes, les boiteries sont courantes et représentent le tiers des visites du vétérinaire praticien. Les maladies podales, responsables ou non de boiterie, sont de plus en plus fréquentes dans le contexte d'élevage intensif actuel. Elles sont d'étiologies diverses, et leur localisation sur l'appareil locomoteur est variable. L'environnement sale et abrasif au niveau des pieds est un facteur fréquemment incriminé comme favorisant l'apparition de maladies dont la conséquence est une boiterie de l'animal. En effet, les sols humides, sales, et les bétons abrasifs facilitent et entretiennent les inflammations de la peau qui sont à l'origine des boiteries observées et de la forte récurrence des maladies.

L'ensemble de ces facteurs stressants contribuant à induire une souffrance et une boiterie chez l'animal est appelé « Stress Abiotique ». Ce stress abiotique induit une abrasion de l'espace interdigité ou des doigts eux-mêmes à l'origine des réactions inflammatoires favorisant l'entrée des bactéries, ou d'autres parasites, responsables des pododermatites et des boiteries. Il fragilise notamment la barrière cutanée et génère un terrain favorable à l'installation et au maintien des affections podales.

Des produits antibiotiques, comme l'oxytétracycline, ainsi que des produits désinfectants pour application en pédiluve existent sur le marché, mais les rythmes et durées d'administration ne sont pas standardisés. Aucune indication n'est donnée quant à la nécessité de les utiliser en fonction du nombre de cas décelés et ces pédiluves ne sont pas toujours très pratiques à mettre en place. Parmi les produits de traitements, certains sont réputés efficaces (formol, sulfate de cuivre), mais risquent d'être prochainement interdits d'utilisation, car ils représentent un risque toxique pour l'homme ou l'environnement. De plus, l'usage des antibiotiques est également critiqué et restreint devant le risque d'apparition de résistance.

On assiste donc à une limitation des moyens disponibles pour contrôler la maladie alors que les mesures mises en œuvre étaient déjà peu aptes à limiter l'expansion des pathologies dont la conséquence est l'apparition d'animaux boiteux.

Aussi, existe-t-il un besoin de fournir des traitements efficaces pour traiter les animaux et préserver leur santé, tout en respectant l'environnement.

Plusieurs approches ont été utilisées pour tenter de diminuer la boiterie des ongulés. Par exemple, la demande française FR3026643A1 décrit l'utilisation d'extraits de plantes aromatiques en combinaison avec des minéraux pour traiter la maladie de Mortellaro chez la vache. Les animaux sont traités en ingérant un complément alimentaire comprenant lesdits extraits ce qui a pour effet de stimuler leurs défenses immunitaires. Un autre exemple est la demande internationale WO1998047374 qui décrit l'utilisation de probiotiques pour la prévention d'infections bactériennes, fongiques ou virales, les probiotiques étant des souches de Bacillus choisies parmi : *Bacillus coagulans, Bacillus subtilis, Bacillus laterosporus* et *Bacillus laevolacticus.* Ces probiotiques sont utilisés pour le traitement de la dermatite et la dermatite de contact. Les probiotiques dont il est question sont appliqués directement sur la peau ou les muqueuses à traiter. Toutefois, aucune démonstration formelle de l'efficacité n'a été montrée.

Aussi, ces méthodes ne semblent pas des plus efficaces et ne montrent pas de solution plus générale aux problèmes de boiterie des ongulés.

Aussi, l'invention vise à pallier ces inconvénients.

Un des buts de l'invention est de proposer un moyen d'améliorer la qualité de vie des animaux atteints de boiterie.

Un autre but de l'invention est de fournir une composition remplissant ces propriétés.

Il est décrit ci-après une composition comprenant un mélange bactérien, pour son utilisation dans le cadre de la prévention et l'amélioration de l'état des ongulés atteints de pathologies impliquant une infection parasitaire ou bactérienne du pied, ladite composition comprenant :
- au moins une souche de bactéries du genre Bacillus, notamment au moins un *Bacillus subtilis,*
- et au moins une souche de bactéries lactiques, notamment au moins un *Lactococcus lactis* et/ou au moins *Pediococcus pentosaceus.* [Page 2bis]

L'invention concerne une composition comprenant un mélange bactérien, pour son utilisation dans le cadre de la prévention et l'amélioration de l'état des ongulés atteints de pathologies impliquant une infection parasitaire ou bactérienne du pied, ladite composition comprenant :
- au moins l'une des quatre souches de *Bacillus subtilis* suivantes : NOL01, NOL02, NOL03 et NOL04, lesdites souches étant respectivement déposées à la CNCM sous le numéro CNCM I - 4606, CNCM I-5043, CNCM I - 4607 et CNCM I - 4608, et
- au moins une bactérie lactique choisie parmi les bactéries lactiques suivantes : *Lactococcus lactis spp lactis 1* souche NOL11 et *Pediococcus pentosaceus 2* souche NOL12, lesdites souches étant respectivement déposées à la CNCM sous le numéro CNCM I - 4609 et CNCM I - 4610.

L'invention repose sur la constatation surprenante faite par les inventeurs que la combinaison de bactéries du genre Bacillus et de bactéries lactiques améliore significativement l'état des ongulés atteints de boiterie causée par une infection parasitaire ou bactérienne du pied.

Dans l'invention, on entend par pathologies impliquant une infection parasitaire ou bactérienne du pied une pathologie dont les symptômes sont liés à une bactérie, mais aussi un virus, un parasite (tel un acarien ou arthropode).

Dans l'invention, comme en zootechnie, on entend par « pied » la partie terminale des quatre membres. Chaque pied comprend deux doigts fonctionnels ; le doigt III, externe ou latéral et le doigt IV, interne ou médial, ainsi que deux doigts accessoires, non fonctionnels, situés en face palmaire du pied, en regard de la deuxième phalange. Ils sont appelés ergots et sont les vestiges des doigts II pour l'interne et V pour l'externe. Le diagnostic différentiel des maladies du pied des bovins ne considèrera ici que les structures anatomiques comprises entre l'articulation métacarpo-phalangienne et l'extrémité des membres postérieurs et antérieurs. On peut aussi parler de région digitale.

Dans l'invention on entend par ongulés les animaux possédant un ou plusieurs sabots à l'extrémité de leurs membres. Le sabot est alors une formation cornée très développée, et qui enveloppe le ou les doigts reposant sur le sol lors de la marche. Les ongulés couverts par la présente invention sont notamment les ongulés domestiques et surtout d'élevage tels que les porcins, les bovins, les caprins, les ovins. Les ongulés préférés sont les porcs, les vaches et les veaux, les chèvres et les moutons, et dans une moindre mesure les chevaux.

Les bactéries du genre Bacillus utilisées dans l'invention sont des bactéries à gram positif appartenant à la famille des bacillacées (Bacillaceae), l'ordre des bacillales (Bacillales), la classe des bacilles (Bacillis), le phyllum des firmicutes (Firmicutes).

Les bactéries lactiques de l'invention sont des bactéries à gram positif.

Les inventeurs ont montré que l'utilisation d'une combinaison d'au moins un bacillus avec au moins une bactérie lactique avait pour effet d'améliorer les conditions de vie des ongulés présentant une boiterie due à une infection podale.

Les différentes souches de l'invention, référencées par leur numéro de dépôt ont toutes été déposée à la Collection Nationale des Cultures de Microorganismes, à l'Institut Pasteur, de Paris (25,28 rue du Docteur Roux 75724 Paris CEDEX 15) selon le traité de Budapest :
- La souche NOL01 a été déposée sous le numéro CNCM I-4606 le 14 mars 2012,
- La souche NOL02 a été déposée sous le numéro CNCM I-5043, le 21 janvier 2016,
- La souche NOL03 a été déposée sous le numéro CNCM I-4607 le 14 mars 2012,
- La souche NOL04 a été déposée sous le numéro CNCM I-4608 le 14 mars 2012,
- La souche NOL11 a été déposée sous le numéro CNCM I-4609 le 14 mars 2012, et
- La souche NOL12 a été déposée sous le numéro CNCM I-4610 le 14 mars 2012.

L'invention concerne l'utilisation d'au moins une souche choisie parmi les souches NOL01, NOL02, NOL03 et NOL04, et au moins une souche choisie parmi les souches NOL11 et Nol12. Aussi, l'invention couvre les 45 combinaisons suivantes :
- NOL01 et NOL11,
- NOL01 et NOL 12,
- NOL01, NOL11 et NOL 12,
- NOL02 et NOL11,
- NOL02 et NOL 12,
- NOL02, NOL11 et NOL 12,
- NOL03 et NOL11,
- NOL03 et NOL 12,
- NOL03, NOL11 et NOL 12,
- NOL04 et NOL11,
- NOL04 et NOL 12,
- NOL04, NOL11 et NOL 12,
- NOL01, NOL02 et NOL11,
- NOL01, NOL02 et NOL12,
- NOL01, NOL02, NOL11 et NOL12,
- NOL01, NOL03 et NOL11,
- NOL01, NOL03 et NOL12,
- NOL01, NOL03, NOL11 et NOL12,
- NOL01, NOL04 et NOL11,
- NOL01, NOL04 et NOL12,
- NOL01, NOL04, NOL11 et NOL12,
- NOL02, NOL03 et NOL11,
- NOL02, NOL03 et NOL12,
- NOL02, NOL03, NOL11 et NOL12,
- NOL02, NOL04 et NOL11,
- NOL02, NOL04 et NOL12,
- NOL02, NOL04, NOL11 et NOL12,
- NOL03, NOL04 et NOL11,
- NOL03, NOL04 et NOL12,
- NOL03, NOL04, NOL11 et NOL12,
- NOL01, NOL02, NOL03 et NOL11,
- NOL01, NOL02, NOL03 et NOL12,
- NOL01, NOL02, NOL03, NOL11 et NOL12,
- NOL01, NOL02, NOL04 et NOL11,
- NOL01, NOL02, NOL04 et NOL12,
- NOL01, NOL02, NOL04, NOL11 et NOL12,
- NOL01, NOL03, NOL04 et NOL11,
- NOL01, NOL03, NOL04 et NOL12,
- NOL01, NOL03, NOL04, NOL11 et NOL12,
- NOL02, NOL03, NOL04 et NOL11,
- NOL02, NOL03, NOL04 et NOL12,
- NOL02, NOL03, NOL04, NOL11 et NOL12,
- NOL01, NOL02, NOL03, NOL04 et NOL11,
- NOL01, NOL02, NOL03, NOL04 et NOL12, et
- NOL01, NOL02, NOL03, NOL04, NOL11 et NOL12.

De manière avantageuse, l'invention concerne une composition pour son utilisation telle que définie ci-dessus, ladite composition comprenant de 10⁵ à 10¹¹ colonies bactériennes de *Bacillus* et de 10⁵ à 10¹¹ colonies bactériennes de bactéries lactiques, les colonies bactériennes étant par mL ou g de composition.

Dans l'invention, de 10⁵ à 10¹¹ colonies bactériennes signifie : environ 10⁵, environ 5.10⁵, environ 10⁶, environ 5.10⁶, environ 10⁷, environ 5.10⁷, environ 10⁸, environ 5.10⁸, environ 10⁹, environ 5.10⁹, environ 10¹⁰, environ 5.10¹⁰ ou environ 10¹¹ colonies bactériennes par mL de culture de bactéries.

L'homme du métier sait aisément comment déterminer ce nombre de bactéries, notamment par comptage soit manuel (en utilisant une lame de Malassez) ou en utilisant un compteur de cellule automatique, ou par dilution puis ensemencement sur gélose et comptage des colonies.

De manière encore plus avantageuse, l'invention concerne une composition pour son utilisation définie ci-dessus, où ladite au moins une souche de Bacillus est sous forme sporulée et/ou sous forme végétative.

Il est notamment particulièrement avantageux que les bactéries du genre Bacillus, et notamment les souches NOL01, NOL02, NOL03 et NOL04 définies ci-dessus soient sous forme sporulées et que les souches de bactéries lactiques soient sous forme végétative.

De manière encore plus avantageuse, l'invention concerne une composition pour son utilisation susmentionnée, dans laquelle lesdites pathologies impliquant une infection parasitaire ou bactérienne du pied des ongulés sont choisies parmi les maladies de Motellaro ou de Fourchet ou encore Tyloma, les panaris ou la Fourbure, ou encore le Piétin, l'Erosion du Bulbe, la nécrose de la pince et la Pododermatite.

La maladie de Montellaro, ou dermatite digitée, a été décrite pour la première fois en 1974 à Milan par Cheli et Mortellaro. Elle a été mise en évidence en France dans les années 1980. Cette maladie semble être de plus en plus fréquente dans les élevages français et pose de nombreuses questions sur sa gestion dans le long terme. On la considère comme la 3ème infection podale en termes de fréquence après l'érosion du talon (fourchet) et les hémorragies de la sole (Bleimes). La maladie de Montellaro est provoquée par *Treponema* et potentiellement par *Fusobacterium necrophorum* Une fois introduite dans un élevage, elle est très rarement éradiquée et réapparaît sous forme de flambées successives. La dermatite digitée est une affection contagieuse caractérisée par une inflammation superficielle circonscrite de la peau de la couronne, qui entraîne des boiteries. Elle touche notamment les bovins. La maladie de Mortellaro est reconnaissable par un pied enflé et une nécrose interdigitée.

La maladie de Fourchet, aussi appelée Le Fourchet, est une infection bactérienne. Elle est causée par une bactérie (provoqué *Dichelobacter nodosus* et *Fusobacterium necrophorum*) qui se plait particulièrement dans un milieu chaud et humide. Le Fourchet, ou dermatite interdigitale, débute par une inflammation humide et malodorante de la peau interdigitale et s'étend à la corne du talon où elle affecte la production cornée. La corne produite est de mauvaise qualité et des fissures apparaissent au niveau du bulbe du talon. Ces fissures provoquent une irritation du pododerme qui réagit en produisant davantage de corne. A partir de ce stade, la situation ne fait que s'aggraver.

Tyloma, ou Limace, est une réaction inflammatoire proliférative de la peau entre les onglons mais il est courant d'avoir des lésions de Mortellaro dessus. La pathologie est donc consécutive à des infections par *Treponema* et *Fusobacterium necrophorum.*

Les panaris ou phlegmons interdigités sont des inflammations aigues des tissus mous caractérisés par une patte enflée et rosée. Ces maladies sont causées par des bactéries, notamment *Arcanobacterium pyogenes et Fusobacterium necrophorum.*

La Fourbure ou pododermatite aseptique diffuse est une lésion au niveau de la sole, qui donne une coloration jaune / rouge pouvant aller jusqu'au bleu, qui peut se développer en ulcère.

Le Piétin est une maladie bactérienne transmise par les pâtures, les litières, les chemins contaminés par des animaux infectés. Cette maladie se caractérise par dans un premier temps une rougeur de l'espace interdigité puis les animaux boitent et présentent des lésions suintantes à l'odeur désagréable. La corne commence ensuite à pourrir, certaines parties de l'onglon se nécrosent et se désolidarisent du pied de l'animal.

L'érosion du bulbe est définie comme la perte irrégulière de la corne du bulbe au niveau du talon, souvent en forme de sillons obliques profonds. Cette infection bactérienne cause de la pourriture au niveau des talons. La nécrose de la pince est une lésion de l'extrémité distale du pied des bovins responsable de boiteries parfois sévères.

La **pododermatite** est une infection bactérienne de la peau généralement située au niveau des ongles ou des sabots des pattes. Chez les ongulés on récence les pathologies suivantes : la pododermatite aseptique (Pododermatitis aseptica acuta, Pododermatitis aseptica chronica), la pododermatite purulente (Pododermatitis purulenta), le chancre ou pododermatite chronique verruqueuse (Pododermatitis chronica verrucosa), certaines formes de gangrène (Pododermatitis gangraenosa), la pododermatite nécrosante (Pododermatitis necrotica), la pododermatite infectieuse (Pododermatitis infectiosa) et la pododermatite hyperplasique chronique (Pododermatitis chronica hyperplastica).

La composition susmentionnée peut donc être utilisée comme décrit ci-dessus pour améliorer les conditions de vie des animaux atteints d'une boiterie causée par les maladies susmentionnées. En effet, le mélange bactérien de l'invention aura un effet limitant la progression des bactéries responsables desdites pathologies.

Le mélange bactérien aura également un effet préventif, en limitant la prolifération des bactéries pathogènes et donc préviendra la boiterie qui en résulte.

Avantageusement, l'invention concerne une composition pour son utilisation telle que définie ci-dessus, ladite composition étant sous forme liquide destinée à être pulvérisée ou à être utilisée dans un bassin.

La composition selon l'invention est notamment proposée sous forme de poudre, mais peut également être proposée sous forme liquide afin d'être appliquée plus facilement sur le pied des ongulés. Cette application sous forme liquide pourra se faire par pulvérisation ou nébulisation directement sur les pieds déjà infectés, ou simplement en prophylaxie sur les pieds sains. Il est également possible de fournir des compositions sous forme liquides placées dans des pédiluves au traverser desquelles les animaux passeront, ce qui mettra en contact la composition de l'invention avec le pied des ongulés.

De manière plus avantageuse, l'invention concerne par ailleurs une composition pour son utilisation susmentionnée, où la dite composition est diluée dans au moins 1 litre de liquide mais au plus 5 litres de liquide pour 50 à 60 animaux.

Il est avantageux, dans le cadre de l'invention, que la composition bactérienne susmentionnée soit diluée dans un volume de liquide de 1 à 5 litres pour traiter de 50 à 60 animaux par pulvérisation.

Par « au moins 1 litre de liquide mais au plus 5 litres » ou « au moins 1 litre de liquide mais au plus 5 litres » on définit dans l'invention que la composition peut être diluée dans 1L, 1,5L, 2L, 2,5L, 3L, 3,5L, 4L, 4,5L ou 5L, lorsque les animaux à traiter sont au nombre de 50 à 60 (soit de 100 à 120 pieds arrières, qui sont les pieds les plus atteints).

Les volumes d'environ 1L sont particulièrement avantageux pour une application notamment par pulvérisation sur le pied. Les volumes plus importants, et notamment 5L sont quant à eux plus appropriés pour des pédiluves. Toutefois, les volumes plus importants peuvent être utilisés en pulvérisation ou nébulisation.

Pour 100 animaux, il est également possible de doubler les volumes susmentionnés de manière proportionnelle, l'homme du métier saura faire les conversions appropriées.

La composition selon l'invention, lorsqu'elle est diluée tel que mentionné ci-dessus, l'est notamment dans un liquide qui est soit de l'eau, soit de l'eau saline (sérum physiologique à 0,9 % en poids de sel par rapport au volume total) ou tout autre liquide physiologiquement acceptable pour une application cutanée ou sur la corne des sabots des animaux.

Le liquide utilisé peut également contenir des additifs physiologiquement acceptables, et notamment du thiosulfate lorsque le liquide est de l'eau. Le thiosulfate permet en effet de neutraliser le chlore contenu dans l'eau courante fournie par les différentes compagnies des eaux. Le liquide peut également contenir des sels et/ou des colorants. Il est important que les additifs n'aient aucune influence sur la vitalité des bactéries contenues dans la composition de l'invention.

Avantageusement, l'invention concerne une composition pour son utilisation susmentionnée, où ladite composition est appliquée sur le pied des ongulés de 1 fois tous les 3 jours à 1 fois tous les 15 jours.

La posologie appropriée pour prévenir la boiterie des animaux ou pour améliorer leurs conditions de vie est une utilisation une fois tous les 3 jours à une fois toutes les deux semaines.

Par « de 1 fois tous les 3 jours à 1 fois tous les 15 jours » on entend dans l'invention une fois tous les 3 jours, une fois tous les 4 jours, une fois tous les 5 jours, une fois tous les 6 jours, une fois tous les 7 jours, une fois tous les 8 jours, une fois tous les 9 jours, une fois tous les 10 jours, une fois tous les 11 jours, une fois tous les 12 jours, une fois tous les 13 jours, une fois tous les 14 jours ou une fois tous les 15 jours.

Il est avantageux de traiter de façon permanente les élevages atteints par les pathologies susmentionnées. Le traitement peut toutefois être modulé à la baisse selon les saisons et l'activité des animaux (notamment si l'animal pâture).

L'invention concerne par ailleurs une composition telle que définie ci-dessus, en association avec au moins un désinfectant, pour une utilisation dans le cadre du traitement des pathologies impliquant une infection parasitaire ou bactérienne du pied des ongulés, ledit au moins un désinfectant étant utilisé au moins 48h avant l'utilisation de ladite composition.

Dans l'invention, il est possible de traiter, préalablement à l'utilisation des compositions susmentionnées, les pieds des ongulés avec des désinfectants. Il est alors impératif que le désinfectant soit arrêté au moins 48h avant l'utilisation de la composition selon l'invention, afin de ne pas tuer les bactéries qu'elle contient.

Les désinfectants utilisés peuvent être ceux communément utilisés par l'homme du métier, à savoir, des antibiotiques (par exemple l'oxytétracycline pour les vaches), le formol, du sulfate de Zn ou de Cu, ou encore de la chaux. D'autres désinfectants connus de l'homme du métier peuvent être utilisés.

L'invention sera mieux comprise à la lumière des quatre exemples et des 11 figures suivantes :

### BREVE DESCRIPTION DES FIGURES

La **Figure 1** est un graphique montrant l'évolution de la note moyenne de l'état des pattes des vaches référentes aux différentes dates de traitement avec la composition selon l'invention.
La **Figure 2** est un histogramme montrant l'évolution de la note moyenne de boiterie des vaches référentes aux différentes dates de traitement avec la composition selon l'invention.
Les **Figures 3A et 3B** sont des diagrammes circulaires montrant la réparation des 4 catégories de boiterie sur les vaches référentes. Les différentes catégories sont les suivantes: en noir: catégorie 0, en blanc : catégorie 1, en gris catégorie 2 et en hachuré : catégorie 3.

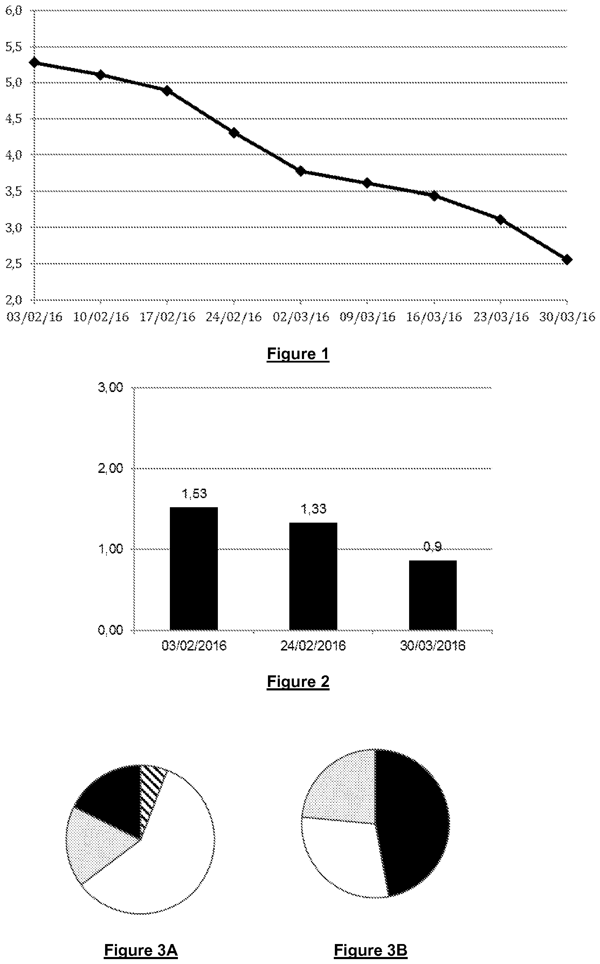

La figure 3A montre les proportions au début de l'essai.
La figure 3B montre les proportions en fin d'essai.

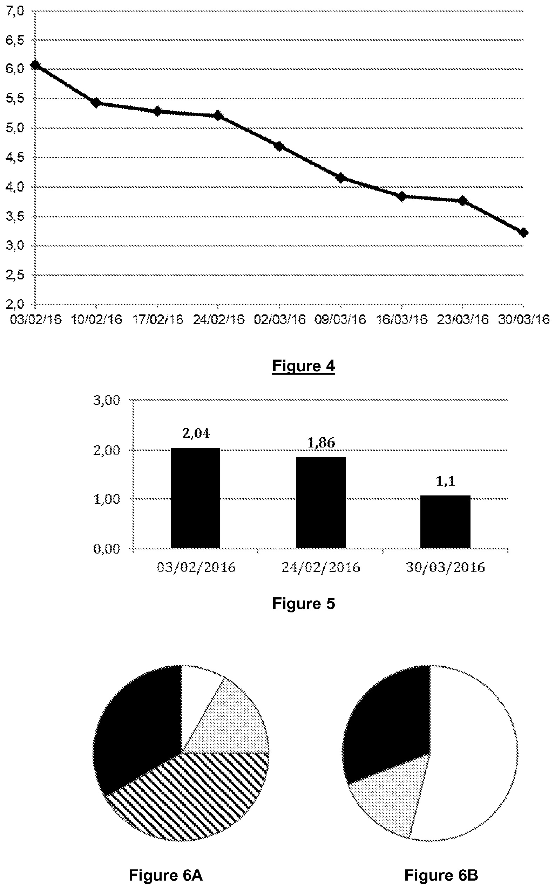

La **Figure 4** est un graphique montrant l'évolution de la note moyenne de l'état des pattes des vaches référentes aux différentes dates de traitement avec la composition selon l'invention.
La **Figure 5** est un histogramme montrant l'évolution de la note moyenne de boiterie des vaches référentes aux différentes dates de traitement avec la composition selon l'invention.
Les **Figures 6A et 6B** sont des diagrammes circulaires montrant la réparation des 4 catégories de boiterie sur les vaches référentes. Les différentes catégories sont les suivantes : en blanc : catégorie 0, en gris : catégorie 1, en hachuré : catégorie 2 et en noir : catégorie 3.
La figure 6A montre les proportions au début de l'essai.
La figure 6B montre les proportions en fin d'essai.

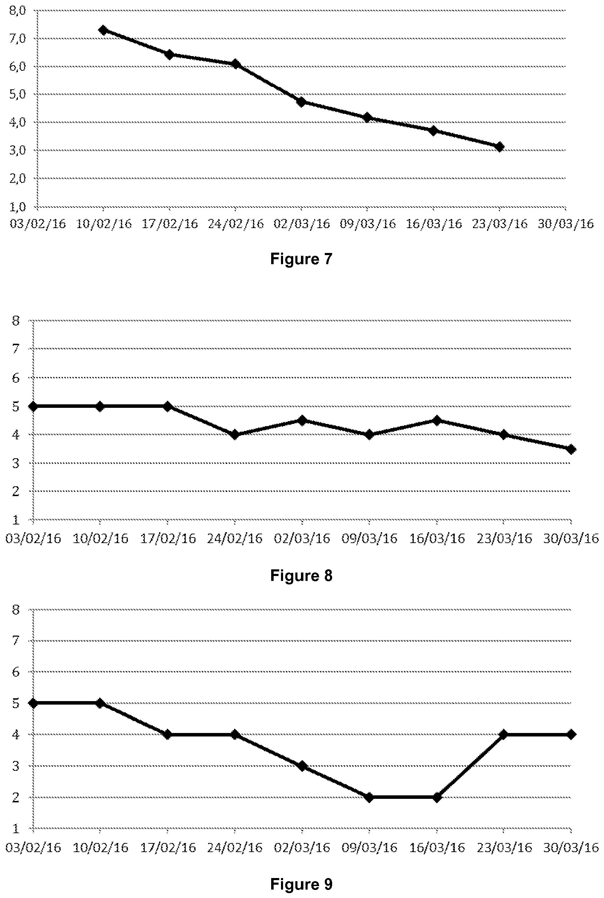

La **Figure 7** est un graphique montrant l'évolution de la note moyenne de l'état des pattes des vaches référentes aux différentes dates de traitement avec la composition selon l'invention.
La **Figure 8** est un graphique montrant l'évolution de la note moyenne de l'état des pattes des vaches du troupeau aux différentes dates de traitement avec la composition selon l'invention.
La **Figure 9** est un graphique montrant l'évolution de la note moyenne de l'état des pattes des vaches du troupeau aux différentes dates de traitement avec la composition selon l'invention.

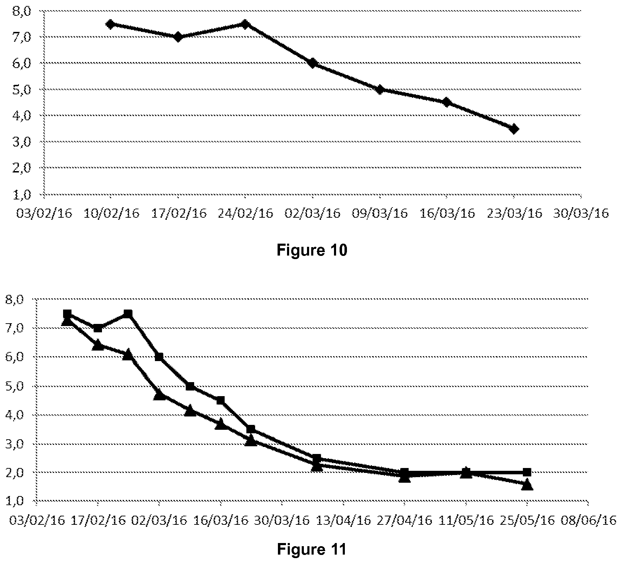

La **Figure 10** est un graphique montrant l'évolution de la note moyenne de l'état des pattes des vaches du troupeau aux différentes dates de traitement avec la composition selon l'invention.
La **Figure 11** est un graphique montrant l'évolution de la note moyenne de l'état des pattes des vaches référentes (courbe avec des triangles) et des vaches du troupeau (courbe avec des carrés) aux différentes dates de traitement avec la composition selon l'invention.

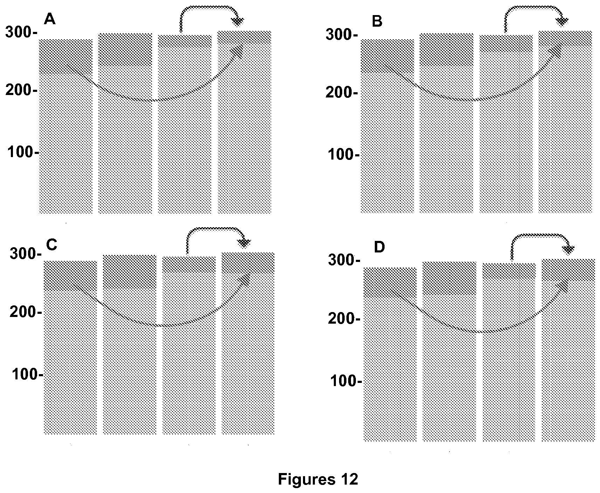

Les **Figures 12A à 12D** représentent des graphiques montrant les proportions de pattes saines (gris clair) et de pattes à problèmes (gris foncé) pour chacune des quatre pattes des brebis, chaque mois après le début du traitement.
La figure 1A montre les résultats pour la patte avant gauche : colonne 1 : janvier - saines 80,1%, problème : 19,9% ; colonne 2 : février - saines 81,4%, problème : 18,6% ; colonne 3 : mars - saines 93,1%, problème : 6,9% ; colonne 1 : avril - saines 92,9%, problème : 7,1%.
La figure 1B montre les résultats pour la patte avant droit: colonne 1 : janvier - saines 80,1%, problème : 19,9% ; colonne 2 : février - saines 81,4%, problème: 18,6% ; colonne 3 : mars - saines 90,3%, problème : 9,7% ; colonne 1 : avril - saines 91,5%, problème : 8,5%.
La figure 1C montre les résultats pour la patte arrière gauche: colonne 1 : janvier - saines 82,6%, problème : 17,4% ; colonne 2 : février - saines 81,1%, problème : 18,9% ; colonne 3 : mars - saines 91%, problème : 9% ; colonne 1 : avril - saines 88,5%, problème : 11,5%.
La figure 1D montre les résultats pour la patte arrière droit: colonne 1 : janvier - saines 82,9%, problème: 17,1% ; colonne 2 : février - saines 81,1%, problème: 18,9% ; colonne 3 : mars - saines 91,3%, problème : 8,7% ; colonne 1 : avril - saines 87,8%, problème : 12,2%.

### EXEMPLES :

### Exemple 1 : préparation des mélanges bactériens

Dans le cadre de l'invention, les inventeurs ont préalablement préparé des flacons renfermant les préparations suivantes :
Préparation 1 = flacon de 20 mL
   - mélange de 4 Bacillus NOL01, NOL02, NOL03 et NOL04, sous la forme sporulée, à une concentration de 10⁵ à 10¹¹ colonies/mL, pour chaque souche bactérienne. Les quatre souches sont mélangées à part égale, dans des proportions respectives 25 :25 :25 :25.
   - Le mélange a été conservé à une température variant de -21 °C à -80°C.
Préparation 2 = flacon de 20 mL
   - des bactéries lactiques NOL11 à une concentration de 10⁵ à 10¹¹ colonies/mL Les bactéries ont été conservées à une température variant de -21 °C à -80°C.

Les inventeurs ont ensuite préparé la composition utilisée pour la suite des expérimentations :
- mélange : 1 flacon de préparation 1 + 1 flacon de préparation 2.

### Exemple 2 : Etude de l'impact d'une application de la composition selon l'invention sur un élevage bovin laitier atteint de Mortellaro

Cet essai fait suite au pré-test qui avait mis en évidence une diminution des cas de Mortellaro dans un élevage suite à une application de la composition de l'invention sur les pattes arrière des vaches. L'objectif de ce nouvel essai est :
- de confirmer les résultats sur 4 nouveaux élevages pour déterminer l'effet de la composition de l'invention sur la problématique de Mortellaro
- de comparer l'effet de la composition avec les traitements préventifs déjà présents dans les élevages
- de mesurer quantitativement l'efficacité de ce traitement

L'essai est réalisé dans 5 élevages de vaches Holstein où le produit a été pulvérisé sur les pattes postérieures des animaux. L'effet de la composition de l'invention a été mesuré par un suivi sanitaire du troupeau (dénombrement et évolution des cas de Mortellaro, traitements vétérinaires).

### I- Caractéristiques des élevages

### Elevage 1 : Normandie (Calvados ; France) intérêt

| | |
|---|---|
| Nombre de vaches | 160/170 |
| Traite | 3 robots Delaval |
| Nombre de lots | 2 |
| Stabulation | Logettes paillées |
| Fréquence de paillage | journalier |
| Quantité de paille | Fort |
| Cornadis | Oui mais pas pour toutes |
| Raclage | Automatique 3 à 4 fois par jour |
| Provenance eau | Forage |
| Traitement eau | Non |
| Traitement actuel Mortellaro | Pédiluve antibiotique + pulvérisation désinfection au robot |

### Elevage 2 : Normandie (Calvados ; France)

| | |
|---|---|
| Nombre de vaches | 190 |
| Traite | Roto extérieur |
| Nombre de lots | 2 |
| Stabulation | Logettes paillées |
| Fréquence de paillage | -- |
| Quantité de paille | Faible (paille ensilée) |
| Cornadis | Non |
| Raclage | Tracteur 2 fois par jour |
| Provenance eau | Réseau de distribution |
| Traitement eau | -- |
| Traitement actuel Mortellaro | Pédiluve antibiobiotique après lavage des pattes pendant la traite |

### Elevage 3 : Normandie (Seine Maritime ; France)

| | |
|---|---|
| Nombre de vaches | 190 + taries |
| Traite | 3 robots Delaval |
| Nombre de lots | 2 (130 vaches pour 2 robots et 60 vaches pour 1 robot) |
| Stabulation | 1er lot en logettes paillées 2nd lot en aire paillée |
| Quantité de paille | Faible moyennement |
| Cornadis | oui |
| Raclage | Automatique |
| Provenance eau | -- |
| Traitement de l'eau | -- |
| Traitement actuel Mortellaro | Pédiluve au formol |

### Elevage 4 : Normandie (Calvados ; France) intérêt

| | |
|---|---|
| Nombre de vaches | 100 |
| Traite | 2 robots |
| Nombre de lots | -- |
| Stabulation | en logettes paillées |
| Quantité de paille | moyennement |
| Cornadis | oui |
| Raclage | Automatique |
| Provenance eau | -- |
| Traitement de l'eau | Traitement contre le chlore |
| Traitement actuel Mortellaro | Pédiluve sec |

### Elevage 5 : Normandie (Calvados ; France)

| | |
|---|---|
| Nombre de vaches | 47+7 taries |
| Traite | 1 robot Delaval |
| Nombre de lots | 1 |
| Stabulation | aires paillées |
| Quantité de paille | -- |
| Cornadis | oui |
| Raclage | -- |
| Provenance eau | Forage |
| Traitement de l'eau | Traitement contre le chlore |
| Traitement actuel Mortellaro | Antibiotiques pulvérisés à la bombe |

### II- Produit utilisé et matériel

Une dose de composition selon l'invention a été préparée à partir de deux flacons distincts de 20 mL :
- Le flacon 1 contenant les Bacillus NOL01, NOL02, NOL03 et NOL04,
- Le flacon 2 contenant les Lactococcus NOL11,
   tels que décrits à l'exemple 1.

Les flacons contiennent les bactéries vivantes dans leur milieu de culture. Ils sont apportés congelés en élevage et doivent être conservés au congélateur dès réception. Le produit est décongelé et dilué dans de l'eau avant nébulisation puis pulvérisé par nébulisation. L'eau utilisée lors de la nébulisation, ne doit pas contenir de chlore, un traitement préalable de l'eau au thiosulfate de sodium peut donc être nécessaire.

L'essai a duré 2 mois avec un traitement réalisé une fois par semaine.

Les traitements des pattes réalisés par les éleveurs avec des composés antibiotiques, ou incompatibles avec les bactéries de la composition selon l'invention, ont été arrêtés au moins 3 jours avant la première application de la composition selon l'invention (fin de semaine 00). La première application du produit sera réalisée en première semaine (début février).

Un état des lieux de l'état sanitaire du troupeau avant le début de l'essai a été réalisé avec l'éleveur. Ce bilan représente le point de départ, référence à partir de laquelle les évolutions sont évaluées. L'état sanitaire du troupeau étant, dans la majorité des élevages de l'essai, stabilisé par un traitement antibiotique ou autre, celui-ci est arrêté dès le début de l'essai.

L'éleveur renseigne ses observations sur l'état sanitaire du troupeau et des relevés qualitatifs seront réalisés par le technicien lors de ses déplacements. Fin des applications du produit en 9^{ème} semaine.

### III- Traitement

Mode d'emploi : à appliquer sous forme liquide. Une dose de produit (constituée des flacons B et L) est nécessaire pour chaque traitement hebdomadaire.

Le traitement se base sur une dose diluée dans 5L d'eau pour traiter les pattes arrière des vaches. La quantité de produit pulvérisé est de l'ordre de 25 mL (soit environ 3 secondes de pulvérisation) par patte soit environ 5L de produit pour traiter les pattes arrières des 100 vaches laitières.

Procédure de préparation de la solution :
- Laisser décongeler le produit à température ambiante entre 15 et 25 °C jusqu'à liquéfaction complète (env. 1h ± 15 minutes)
- Préparer 5L d'eau dans le réservoir du nébulisateur et ajouter 80mg de thiosulfate de sodium ou 10mL si solution liquide (Si l'eau est traitée au peroxyde d'hydrogène, utiliser impérativement de l'eau minérale ou de l'eau non traitée).
- Enlever la capsule et retirer le bouchon des flacons
- Verser les deux flacons du produit dans l'eau traitée et ajouter environ 10 mL de colorant E 133 (colorant bleu brillant de qualité alimentaire).
- Mélanger pour homogénéiser le produit puis laisser reposer pendant 15 minutes
- Pulvériser les pattes arrière des vaches de l'ordre de 25mL par patte
- Ce traitement est à renouveler toutes les semaines de l'essai (9 traitements au total).

| | **Elevage 1** | **Elevage 2** | **Elevage 3** | **Elevage 4** | **Elevage 5** |
|---|---|---|---|---|---|
| Nombre de doses par application | 2 | 2 | 2 | 1 | 1 |
| Quantité d'eau | 10L | 10L | 10L | 5L | 5L(seul 2,5L sont pulvérisés) |
| Quantité de thiosulfate ajouté | 160mg (2 doses) | 160mg (2 doses) | 160mg (2 doses) | 80mg (1 dose) | 80mg (1 dose) |
| Quantité de colorant ajouté | 8mL | Pas de colorant | 8mL | 4mL | 4mL |

Pour l'élevage 5, une solution d'une dose diluée dans 5L normalement prévue pour 100 vaches est préparée, mais seule la moitié est utilisée pour l'application, le reste est jeté.

### IV- Description des mesures et des analyses

### 1- Mesures communes pour l'ensemble des élevages

### Notation de l'état de chaque vache référence :

Le premier jour de l'essai, une dizaine de vaches identifiées par l'éleveur comme très atteintes ou particulièrement vulnérables à la problématique Mortellaro seront suivies plus précisément. Une notation indicative de l'état de chaque vache sera donnée par l'éleveur sur une échelle de 0 à 10 : 0 représentant une vache saine et 10 une vache gravement atteinte.

### 2- Notation globale de l'état du troupeau :

L'éleveur donnera également une note globale de l'état de son troupeau sur une échelle de 0 à 10 avec : 0 pour un troupeau totalement sain et 10 pour 100% des animaux atteints.

### 3- Photos des pattes des vaches, application :

Des photos des pattes arrière des vaches seront réalisées pour donner un visuel des observations possibles en élevage et des signes cliniques des cas de Mortellaro sans lever les pattes. Des photos de la préparation et de l'application seront également prises.

### 4- Notation boiterie des vaches références :

Une notation de boiterie est réalisée sur les vaches références selon la grille suivante :
0 : Bonne mobilité - Marche avec une répartition uniforme du poids et du rythme sur les quatre pattes avec un dos plat. La vache peut réaliser des foulées longues et fluides.
1 : Mobilité imparfaite - Marche non homogène (sur la répartition du poids ou du rythme) ou réduite ; affecte une ou plusieurs pattes, difficilement identifiable.
2 : Mobilité réduite - Marche non homogène sur une patte facilement identifiable et/ou des foulées nettement réduites (en général associée à un dos arqué).
3 : Mobilité sévèrement réduite - Incapable de marcher aussi vite qu'un homme à vive allure (ne peut pas suivre le troupeau en bonne santé) et présente des signes du score 2 ci-dessus.

### 5- Mesure spécifique pour l'élevage 2

### Notation de la réaction des vaches lors du lavage :

Une notation de la réaction des vaches sera réalisée lors du lavage des pattes. Les lésions présentes lors d'un stade aigu de Mortellaro sont très sensibles, le jet d'eau lors du lavage des pattes atteintes provoque donc souvent une réaction des vaches qui lèvent ou bougent leurs pattes. Cette réaction sera donc relevée pour chacune des pattes arrières par la notation suivante :
Absence de réaction - Réaction faible - Réaction forte

### 6- Mesure spécifique pour l'élevage 5

### Photos des pattes arrière de l'ensemble des vaches :

Dans cet élevage, l'ensemble du troupeau passera dans une cage de parage (louée ou intervention d'un pareur) et une photo de chaque patte arrière sera réalisée. A l'aide de ces photos, une notation de chaque patte sera réalisée selon la grille de notation suivante :
M0 : sain. Vache saine sans lésion de dermatite digitée
M1 : stade débutant. Lésion bien circonscrite, aspect « peau de fraise », rose ou gris, inférieure à 2 cm. Généralement douloureuse.
M2 : stade aigu. Lésions qui font boiter les vaches car hyper sensibles et douloureuses. Ulcérations de la peau localisées au bord de la corne du sabot (1) ou autour des onglons (2). Evolution : inflammation chronique et prolifération de la peau en petits nodules surélevés (3) ou en filaments (poils géants) (4). Souvent recouvertes de pus à l'odeur fétide.
M3 : stade de cicatrisation. Apparaît après un traitement efficace, la lésion se recouvre d'une croûte noire et devient insensible. La guérison peut être total (retour au stade M0) ou partielle (stade M4)
M4 : réservoir de la maladie. Lésion chronique non active, sèche et peu sensible. Peau épaisse avec prolifération de poils géants
M4-1 : Forme M1 active sur lésion M4, souvent sans rebords nets.

### 7- Relevés réalisés par l'éleveur

Si l'éleveur réalise déjà des relevés sur des problématiques de boiteries de manière régulière, elles seront récupérées et utilisées pour évaluer l'effet du produit (ex : nombre de vaches boiteuses, traitement individuel, réforme de vache pour cette cause...).

Le ressenti de l'éleveur sur l'effet global du traitement et toute observation ou remarques sont également relevés.

De même, si l'éleveur possède un historique des fréquences de Mortellaro / boiteries ou des traitements vétérinaires, ces données sont utilisées pour avoir un point de comparaison avec la période de l'essai.

### V- Résultats

### Elevage 2

### Résultat pour l'élevage 2 : notation de l'état de la patte

| | **Notation état des pattes sur une échelle de 0 à 10 (0 = RAS, 10 = très gros soucis de pattes)** | | | | | | | | |
|---|---|---|---|---|---|---|---|---|---|
| **N° de la vache** | 03/02/16 | 10/02/16 | 17/02/16 | 24/02/16 | 02/03/16 | 09/03/16 | 16/03/16 | 23/03/16 | 30/03/16 |
| 8480 | 0 | 0 | 0 | 0 | 0 | 0 | 0 | 0 | 0 |
| 6264 | 7 | 7 | 6 | 5 | 5 | 5 | 5 | 4 | 2 |
| 5959 | 4 | 4 | 3 | 3 | 2,5 | 2 | 2 | 1 | 1 |
| 6300 | 9 | 9 | 9 | 8 | 7 | 6 | 6 | 5 | 5 |
| 6271 | 9 | 9 | 9 | 8 | 8 | 8 | 8 | 8 | 8 |
| 6367 | 4 | 4 | 3 | 3 | 3 | 4 | 5 | 5 | 4,5 |
| 6244 | 7 | 7 | 7 | 6 | 6 | 6 | 5 | 5 | 5 |
| 6209 | 4 | 4 | 4 | 3 | 2 | 1 | 1 | 1 | 0 |
| 6242 | 9 | 9 | 9 | 8,5 | 7 | 7 | 7 | 7 | 7 |
| 4834 | 8 | 6 | 5 | 5 | 4 | 5 | 5 | 4 | 2 |
| 8535 | 4 | 4 | 4 | 3 | 2 | 2 | 2 | 1,5 | 1 |
| 6010 | 4 | 5 | 5 | 5 | 4 | 4 | 3 | 2 | 2 |
| 0750 | 4 | 4 | 3 | 3 | 2 | 2 | 1 | 1 | 1 |
| 6289 | 6 | 5 | 5 | 4 | 4,5 | 4 | 4 | 4,5 | 4 |
| 4837 | 4 | 4 | 4 | 3 | 2 | 2 | 2 | 1 | 1 |
| 6232 | 4 | 3 | 3 | 2 | 2 | 2 | 2 | 2 | 0 |
| 5993 | 4 | 4 | 3 | 2 | 2 | 1 | 1 | 1 | 0 |
| 6416 | 4 | 4 | 6 | 6 | 5 | 4 | 3 | 3 | 2,5 |

On observe une amélioration moyenne de 2,7 points pour les vaches à problème, avec 16 vaches sur 18 qui ont baissé en boiteries. Les 2 autres ont un état stabilisé

L'évolution de la note moyenne au cours de l'essai est représentée en Figure 1.

### Résultats sur la boiterie : notation de la boiterie

| | **Notation de boiterie entre 0 et 3 (cf grille)** | | |
|---|---|---|---|
| **N° de la vache** | 03/02/16 | 24/02/16 | 30/03/16 |
| 8480 | 0 | 0 | 0 |
| 6264 | 2 | 1 | 1 |
| 5959 | 1 | 0 | 0 |
| 6300 | 2,5 | 3 | 2,5 |
| 6271 | 3 | 2 | 2 |
| 6367 | 1 | 1 | 1 |
| 6244 | 2 | 1 | 2 |
| 6209 | 1 | 0 | 0 |
| 6242 | 3 | 3 | 2 |
| 4834 | 3 | 2 | 0 |
| 8535 | 1 | 1 | 1 |
| 6010 | 1 | 2 | 1 |
| 0750 | 1 | 1 | 0 |
| 6289 | 2 | 2 | 2 |
| 4837 | 1 | 2 | 0 |
| 6232 | 1 | 0 | 0 |
| 5993 | 1 | 1 | 0 |
| 6416 | 1 | 2 | 1 |

A la fin de l'essai, 11 vaches sur 18 ont baissé de catégorie de boiterie, et aucune aggravation sur les vaches suivies n'a été observée. Les résultats sont montrés en **Figures 2 et 3A à 3B****.**

Les résultats sur cet élevage sont très favorables.

Avant le traitement avec la composition selon l'invention, les vaches recevaient une fois par semaine le traitement suivant :
1. Lavage des pattes des vaches en salle de traite
2. Pédiluves de formol, sulfate de Cu et sulfate de Zn avec un changement du pédiluve entre les 2 lots

Pendant l'essai, les animaux ont été traités de la manière suivante une fois par semaine :
1- Lavage des pattes des vaches en salle de traite (seulement la 1ère moitié de l'essai)
2- Application de la composition selon l'invention en salle de traite au pulvérisateur.

Le traitement selon l'invention a permis de passer de 7 à 8 traitements à l'oxytétracycline par semaine à un traitement de 1 ou trois durant la même période. Alors que l'odeur d'infection des vaches était très présente avant l'essai, cette odeur a été largement diminuée.

Selon l'éleveur, sans le lavage des pattes en salle de traite, l'application des flores avec le matériel utilisé est moins contraignante que le système pédiluve

Concernant le parage, l'ensemble du troupeau a subi un parage 2 semaines avant la fin de l'essai sans informer les pareurs de l'essai. Les pareurs ont conclu que c'était la première année où ils n'ont pas mentionné de problèmes de dermatites (contamination < à 5% du troupeau).

### Elevage 3

### Résultat pour l'élevage 3 : notation de l'état de la patte

| | **Notation état des pattes sur une échelle de 0 à 10 (0 = RAS, 10 = très gros soucis de pattes)** | | | | | | | | |
|---|---|---|---|---|---|---|---|---|---|
| **N° de la vache** | 03/02/16 | 10/02/16 | 17/02/16 | 24/02/16 | 02/03/16 | 09/03/16 | 16/03/16 | 23/03/16 | 30/03/16 |
| 1758 | 10 | 8 | 5 | 5 | 5 | 5 | 5 | 10 | 10 |
| 0751 | 5 | 3 | 2 | 1 | 1 | 0 | 0 | 0 | 0 |
| 9086 | 6 | 3 | 2 | 2 | 2 | 0 | 0 | 0 | 0 |
| 9152 | 6 | 3 | 3 | 3 | 2 | 0 | 0 | 0 | 0 |
| 5376 | 2 | 2 | 2 | 2 | 2 | 3 | 2 | 1 | 0 |
| 0227 | 5 | 5 | 5 | 5 | 4 | 3 | 2 | 2 | 0 |
| 0226 | 5 | 8 | 8 | 8 | 7 | 7 | 7 | 8 | 8 |
| 9144 | 8 | 5 | 8 | 8 | 8 | 7 | 7 | 7 | 8 |
| 3589 | 8 | 8 | 8 | 8 | 10 | 10 | 9 | 7 | 2 |
| 0216 | 8 | 8 | 8 | 8 | 8 | 8 | 8 | 8 | 8 |
| 0366 | 6 | 5 | 5 | 5 | 4 | 4 | 3 | 0 | 0 |
| 9460 | 8 | 7 | 7 | 7 | 7 | 6 | 6 | 5 | 6 |
| 0294 | 2 | 2 | 2 | 2 | 1 | 1 | 1 | 1 | 0 |
| 1787 | | 9 | 9 | 9 | 6 | 5 | 5,5 | 8 | 8 |

On observe une amélioration moyenne de 2,9 points pour les vaches à problème. 11 vaches sur 14 ont un état amélioré de leurs pattes sauf les 2 dernières semaines. En effet, les deux dernières semaines, un souci de racleur a entrainé une dégradation de la propreté des zones de vie ce qui a probablement augmenté la pression bactérienne environnante et s'est répercutée sur l'état de 2 vaches références.

L'évolution de la note moyenne au cours de l'essai est représentée en **Figure 4****.**

### Résultats sur la boiterie : notation de la boiterie

| | **Notation de boiterie entre 0 et 3 (cf grille)** | | |
|---|---|---|---|
| **N° de la vache** | 03/02/16 | 24/02/16 | 30/03/16 |
| 1758 | 3 | 2 | 3 |
| 0751 | 3 | 1 | 0 |
| 9086 | 1 | 1 | 0 |
| 9152 | 2 | 1 | 0 |
| 5376 | 2 | 2 | 1 |
| 0227 | 0 | 0 | 0 |
| 0226 | 2 | 3 | 3 |
| 9144 | 2 | 2 | 3 |
| 3589 | 1 | 2 | 0 |
| 0216 | 3 | 3 | 3 |
| 0366 | 3 | 2 | 0 |
| 9460 | 2,5 | 2 | 1 |
| 0294 | 2 | 2 | 0 |
| 1787 | | 3 | 3 |

A la fin de l'essai, 9 vaches sur 14 ont une meilleure mobilité, 3 n'ont pas évolué et 2 se sont dégradées. On note toutefois une amélioration d'une catégorie sur l'ensemble des vaches références. Les résultats sont montrés en **Figures 5 et 6A à 6B**.

Les résultats sur cet élevage sont également très favorables.

Avant le traitement avec la composition selon l'invention, les vaches recevaient comme traitement des pédiluves au formol.

Pendant l'essai, les animaux ont été traités de la manière suivante une fois par semaine au cornadis par application de la composition selon l'invention au pulvérisateur.

Selon l'éleveur, avant traitement, de nombreux cas de boiteries apparaissaient régulièrement. Après traitement, seules 2 à 3 vaches ont présenté des problèmes, et seules quelques vaches n'ont pas bien répondu à la composition.

L'éleveur est convaincu de l'efficacité de la composition alors qu'il était sceptique avant l'essai.

### Elevage 5

### Résultat pour l'élevage 5 : notation de l'état de la patte

| | **Notation état des pattes sur une échelle de 0 à 10 (0 = RAS, 10 = très gros soucis de pattes)** | | | | | | |
|---|---|---|---|---|---|---|---|
| **N° de la vache** | 10/02/16 | 17/02/16 | 24/02/16 | 02/03/16 | 09/03/16 | 16/03/16 | 23/03/16 |
| 5722 | 10 | 9 | 9 | 9 | 9 | 9 | 9 |
| 2095 | 8 | 7 | 6 | 5 | 4 | 4 | 3 |
| 5377 | 8 | 8 | 7 | 5 | 4 | 3 | 3 |
| 5794 | 8 | 8 | 7 | 5 | 5 | 5 | 5 |
| 2105 | 7 | 6 | 6 | 4 | 3 | 3 | 2 |
| 5795 | 8 | 6 | 6 | 5 | 4 | 3 | 2 |
| 5801 | 10 | 7,5 | 7 | 6 | 6 | 6 | 5 |
| 5728 | 7 | 6 | 6 | 6 | 6 | 5 | 3 |
| 5788 | 7 | 6 | 5 | 4 | 4 | 3 | 3 |
| 1937 | 6,5 | 6 | 5 | 3,5 | 3 | 2 | 1 |
| 2082 | 7 | 7 | 6 | 4,5 | 3 | 3 | 2 |
| 1826 | 8 | 7 | 6 | 6 | 6 | 5 | 5 |
| 2077 | 7 | 6 | 6 | 4,5 | 3 | 2,5 | 2 |
| 5581 | 4 | 4 | 3,5 | 2 | 1,5 | 1 | 1 |
| 2029 | 4 | 3 | 2 | 1,5 | 1 | 1 | 1 |
| 2108 | | | 10 | 8 | 6 | 4 | 3 |

On observe une amélioration moyenne de 4.2 points pour les vaches à problème, avec 15 vaches sur 16 qui une amélioration de l'état de leurs pattes. Une seule vache est restée en mauvais état.

L'évolution de la note moyenne au cours de l'essai est représentée en **Figure 7****.**

Les résultats sur cet élevage sont ici encore très favorables.

Avant le traitement avec la composition selon l'invention, les vaches recevaient une application de désinfectants au robot (4Hooves).

Pendant l'essai, les animaux ont été traités de la manière suivante une fois par semaine au cornadis par application de la composition selon l'invention au pulvérisateur.

Avant le traitement, les vaches fortement atteintes étaient traitées individuellement avec de l'oxytétracycline. Aucun de ces traitements n'a été nécessaire après application de la composition selon l'invention.

Selon l'éleveur, il y a une bonne amélioration du troupeau, et bien moins de vaches doivent être accompagnées jusqu'en salle de traite.

Elevage 1 : l'éleveur a souhaité arrêter l'essai, car il devait stopper ses anciennes pratiques (désinfection journalière alternée toutes les semaines entre peroxyde et eau de javel) et a vu une dégradation de l'état de son troupeau après une seule semaine.

Elevage 3 : l'essai a été arrêté car les inventeurs se sont aperçus que l'éleveur avait changé d'asséchant sur ses logettes en passant d'un asséchant classique à de la chaux éteinte appliquée matin et soir. La chaux éteinte ayant un pouvoir désinfectant, une incompatibilité avec la composition de l'invention a donc été soulevée. L'éleveur a fait le choix d'arrêter l'essai plutôt que d'arrêter la chaux.

Les **Figures 8 à 10** montrent les évolutions globales des troupeaux dans les 3 élevages.

Ces résultats, suite à une application de la composition selon l'invention, ne sont visibles qu'environ 3 semaines après la première application, temps nécessaire à l'implantation des bactéries contenues dans la composition selon l'invention.

L'arrêt d'un ancien traitement régulier qui stabilisait l'état du troupeau risque d'entrainer une dégradation les 2 premières semaines du traitement avec la composition selon l'invention. Un traitement individuel de chaque vache peut alors être nécessaire.

### Exemple 3 : Test de réduction de la fréquence d'application

Les inventeurs ont voulu tester si une diminution de la fréquence d'application à 1 fois toutes les 2 semaines permet de conserver une situation stabilisée après 2 mois d'application hebdomadaire.

Pour se faire, le protocole décrit à l'exemple précédent a été légèrement modifié. Pour l'élevage 2, à partir du 30/03/2016, l'éleveur est passé à une application toutes les 2 semaines avec un dosage équivalent (2 doses pour 100 vaches utilisées pour les 190 vaches du troupeau).

Pour l'élevage 5, à partir du 15/04/2016, l'éleveur est passé à une application toutes les 2 semaines avec un dosage équivalent (1 dose pour 100 vaches pour les 55 vaches du troupeau).

### Elevage 3 :

L'état des vaches référentes continue de s'améliorer et se stabilise à un bon état des pattes à la suite de la réduction du traitement. En outre l'état général du troupeau se stabilise à un bon état des pattes.

Depuis le mois d'avril, les vaches sortent en balade.

Le passage d'une application toutes les semaines à une application toutes les deux semaines stabilise la situation globale du troupeau à un bon état sur la problématique des pattes. Aucune dégradation n'a été observée.

Les résultats sont montrés dans la **Figure 11****.**

Le tableau suivant montre les valeurs obtenues :

| **N° de la vache** | 10/02/16 | 17/02/16 | 24/02/16 | 02/03/16 | 09/03/16 | 16/03/16 | 23/03/16 | 07/04/16 | 27/04/16 | 11/05/16 | 25/05/16 |
|---|---|---|---|---|---|---|---|---|---|---|---|
| 5722 | 10 | 9 | 9 | 9 | 9 | 9 | 9 | 9 | 7 | 7 | 6 |
| 2095 | 8 | 7 | 6 | 5 | 4 | 4 | 3 | 2 | 2 | 2 | 2 |
| 5377 | 8 | 8 | 7 | 5 | 4 | 3 | 3 | 3 | 3 | 3 | 2 |
| 5794 | 8 | 8 | 7 | 5 | 5 | 5 | 5 | 4 | 2 | 2 | 2 |
| 2105 | 7 | 6 | 6 | 4 | 3 | 3 | 2 | 1 | 1 | 2 | 1 |
| 5795 | 8 | 6 | 6 | 5 | 4 | 3 | 2 | 1 | 1 | 1 | 1 |
| 5801 | 10 | 7,5 | 7 | 6 | 6 | 6 | 5 | 4 | 3 | 3 | 2 |
| 5728 | 7 | 6 | 6 | 6 | 6 | 5 | 3 | 2 | 2 | 3 | 2 |
| 5788 | 7 | 6 | 5 | 4 | 4 | 3 | 3 | 2 | 2 | 2 | 2 |
| 1937 | 6,5 | 6 | 5 | 3,5 | 3 | 2 | 1 | 0 | 0 | 0 | 0 |
| 2082 | 7 | 7 | 6 | 4,5 | 3 | 3 | 2 | 1 | 1 | 1 | 1 |
| 1826 | 8 | 7 | 6 | 6 | 6 | 5 | 5 | 4 | 3 | 3 | 2 |
| 2077 | 7 | 6 | 6 | 4,5 | 3 | 2,5 | 2 | 1 | 1 | 1 | 1 |
| 5581 | 4 | 4 | 3,5 | 2 | 1,5 | 1 | 1 | 0 | 0 | 0 | 0 |
| 2029 | 4 | 3 | 2 | 1,5 | 1 | 1 | 1 | 0 | 0 | 0 | 0 |
| 2108 | | | 10 | 8 | 6 | 4 | 3 | 3 | 2 | 2 | 2 |

### Pour l'élevage 1 :

Après une phase de transition qui a nécessité des traitements antibiotique suivi d'un protocole d'application renforcé de la composition de l'invention (pattes + bâtiment), l'état général du troupeau s'est considérablement amélioré.

L'éleveur a selon ses dires retrouvé une situation qu'il ne connaissait plus depuis un an et demie. Il a essayé d'espacer un peu les applications en passant à 10/12 jours, mais il a bien observé qu'il ne fallait pas aller au-delà de 12 jours, car les pattes commencent à "rosir", signe d'une réapparition des symptômes.

### Pour l'élevage 5 :

On observe une forte dégradation de l'état sanitaire de l'élevage suite à un mauvais entretien de la stabulation, suite à une erreur humaine.

L'élevage a été repris en main et une application renforcée des applications sur les pattes et l'environnement a été réalisé pendant 1 mois avec la composition selon l'invention, pour rétablir l'état sanitaire du troupeau

L'état actuel de l'élevage est meilleur, et le parage du troupeau a démarré.

Dans une période où les vaches ont un accès au pâturage, la pression pathogène est moins forte (moins de fréquentation de zones souillées), une application toutes les 2 semaines ou plus n'engendre pas de dégradation de l'état des pattes du troupeau.

### Exemple 4 : Etude de l'impact d'une application de la composition selon l'invention sur un élevage ovin

Dans cet exemple, les inventeurs ont voulu tester l'efficacité de la composition selon l'invention pour le traitement ou l'amélioration de la boiterie chez les brebis, dans le cadre du Piétin.

L'essai a été réalisé sur un élevage de 300 brebis de la race Lacaune dans un élevage des Pyrénées-Atlantiques.

Les brebis ont été traitées avec la composition selon l'invention par une application des flores 1 fois par semaine pendant 12 semaines par tapis pédiluve. Lors de chaque journée d'application, les brebis passent 2 fois sur le tapis (1 fois par traite).

Les bactéries constituant la composition selon l'invention sont mise en œuvre à raison d'une dose représentant 40mL de la composition comme décrit dans l'exemple 1, dans 30L d'eau avec un colorant bleu brillant E133 et du thiosulfate, puis la solution est versée dans le pédiluve.

### Résultats

Les résultats globaux sont présentés dans le tableau suivant :

| | Janvier | Février | Mars | Avril |
|---|---|---|---|---|
| Nombre de brebis | 281 | 291 | 288 | 295 |
| Brebis à problème | 91 | 106 | 94 | 113 |
| Pas problème | 190 | 185 | 194 | 182 |
| 1 patte | 5 | 0 | 89 | 110 |
| 2 pattes | 69 | 103 | 5 | 3 |
| 3 pattes | 3 | 0 | 0 | 0 |
| 4 pattes | 14 | 3 | 0 | 0 |

On constate que les proportions sont les suivantes :

| | Janvier | Février | Mars | Avril |
|---|---|---|---|---|
| Pas problème | 68% | 64% | 67% | 62% |
| 1 patte | 2% | 0% | 31% | 37% |
| 2 pattes | 25% | 35% | 2% | 1% |
| 3 pattes | 1% | 1% | 0% | 0% |
| 4 pattes | 5% | 0% | 0% | 0% |

Test du Khi deux entre les dates 2 à 2 sur la distribution des brebis par nombre de patte à problème :

| **p-value** | janvier | février | mars | avril |
|---|---|---|---|---|
| janvier | / | | | |
| février | **2,26E-04** | / | | |
| mars | **2,20E-16** | **2,20E-16** | / | |
| avril | **2,20E-16** | **2,20E-16** | **0,2214** | / |

Les distributions sont significativement différentes entre les dates sauf entre mars et avril

Les brebis à problème le premier jour de l'essai se sont quasiment toutes améliorées sur leur nombre de patte à problème.

Toutefois, une dégradation est possible dans les premières semaines suite à l'arrêt d'un traitement antérieur.

Entre le début et la fin de l'essai, sur les 82 brebis à problème présentes sur la totalité de l'essai, 79 ont baissé de catégorie. Les brebis à problème en fin d'essai sont de nouvelles brebis

Les résultats pattes par pattes sont présentés aux figures 12A à 12D.

Ces résultats montrent que 96% des brebis à problème en début d'essai et présentes sur la totalité de l'essai ont eu au moins 1 patte de moins à problème après le traitement.

De plus à l'issue de l'essai, il n'y a pas de brebis avec 2 pattes ou plus à problème.

Collectivement, ces résultats montrent l'efficacité de la composition selon l'invention sur la problématique de boiterie.

L'invention n'est pas limitée aux modes de réalisation présentés et d'autres modes de réalisation apparaîtront clairement à l'homme du métier.

## Revendications

1. Composition comprenant un mélange bactérien, pour son utilisation dans le cadre de la prévention et l'amélioration de l'état des ongulés atteints de pathologies impliquant une infection parasitaire ou bactérienne du pied, ladite composition comprenant :
- au moins l'une des quatre souches de *Bacillus subtilis* suivantes : NOL01, NOL02, NOL03 et NOL04, lesdites souches étant respectivement déposées à la CNCM sous le numéro CNCM I - 4606, CNCM I-5043, CNCM I - 4607, CNCM I - et CNCM I - 4608, et
- au moins une bactérie lactique choisie parmi les bactéries lactiques suivantes : *Lactococcus lactis spp lactis 1* souche NOL11 et *Pediococcus pentosaceus 2* souche NOL12, lesdites souches étant respectivement déposées à la CNCM sous le numéro CNCM I - 4609 et CNCM I - 4610.

2. Composition pour son utilisation selon la revendication 1, ladite composition comprenant de 10⁵ à 10¹¹ colonies bactériennes de *Bacillus* et de 10⁵ à 10¹¹ colonies bactériennes de bactéries lactiques, les colonies bactériennes étant par mL ou g de composition.

3. Composition pour son utilisation selon la revendication 1 ou la revendication 2, où ladite au moins une souche de Bacillus est sous forme sporulée et/ou sous forme végétative.

4. Composition pour son utilisation selon l'une quelconque des revendications 1 à 3, dans laquelle lesdites pathologies impliquant une infection parasitaire ou bactérienne du pied des ongulés sont choisies parmi les maladies de Motellaro ou de Fourchet ou encore Tyloma, les panaris ou la Fourbure, ou encore le Piétin, l'Erosion du Bulbe, la nécrose de la pince et la Pododermatite.

5. Composition pour son utilisation selon l'une quelconque des revendications 1 à 4, ladite composition étant sous forme liquide destinée à être pulvérisée ou à être utilisée dans un bassin.

6. Composition pour son utilisation selon l'une quelconque des revendications 1 à 5, où ladite composition est diluée dans au moins 1 litre à au plus 5 litres de liquide pour 50 à 60 animaux.

7. Composition pour son utilisation selon l'une quelconque des revendications 1 à 6, où ladite composition est appliquée sur le pied des ongulés de 1 fois tous les 3 jours à 1 fois tous les 15 jours.

8. Composition telle que définie dans l'une quelconque des revendications 1 à 7, en association avec au moins un désinfectant, pour une utilisation dans le cadre du traitement des pathologies impliquant une infection parasitaire ou bactérienne du pied des ongulés,
ledit au moins un désinfectant étant utilisé au moins 48h avant l'utilisation de ladite composition.

## Patentansprüche

1. Zusammensetzung, umfassend eine Bakterienmischung, zum Verwenden derselben bei der Vorbeugung und Verbesserung des Zustands von Huftieren mit Pathologien, die eine parasitäre oder bakterielle Infektion des Fußes beinhalten, wobei die Zusammensetzung umfasst:
- mindestens einen der folgenden vier Stämme von *Bacillus subtilis:* NOL01, NOL02, NOL03 und NOL04, wobei die Stämme jeweils bei der CNCM (Collection Nationale de Cultures de Microorganismes) unter den Nummern CNCM I-4606, CNCM I-5043, CNCM I-4607, CNCM I- und CNCM I-4608 hinterlegt sind, und
- mindestens ein Milchsäurebakterium, ausgewählt aus den folgenden Milchsäurebakterien: *Lactococcus lactis spp lactis* 1 Stamm NOL11 und *Pediococcus pentosaceus* 2 Stamm NOL12, wobei die Stämme bei der CNCM unter der Nummer CNCM I-4609 bzw. CNCM 1-4610 hinterlegt sind.

2. Zusammensetzung zum Verwenden derselben nach Anspruch 1, wobei die Zusammensetzung 10⁵ bis 10¹¹ Bakterienkolonien von *Bacillus* und 10⁵ bis 10¹¹ Bakterienkolonien von Milchsäurebakterien umfasst, wobei die Bakterienkolonien einem ml oder g der Zusammensetzung entsprechen.

3. Zusammensetzung zum Verwenden derselben nach Anspruch 1 oder 2, wobei der mindestens eine Bacillus-Stamm in Sporenform und/oder in vegetativer Form vorliegt.

4. Zusammensetzung zum Verwenden derselben nach einem der vorhergehenden Ansprüche 1 bis 3, wobei die Pathologien, die eine parasitäre oder bakterielle Infektion des Fußes von Huftieren beinhalten, aus der Motellaro'schen oder Fourchet'schen-Krankheiten oder Tyloma, Panariasis oder Laminitis oder Fußfäule, Ballenerosion, Sohlenspitzennekrose und Pododermatitis ausgewählt sind.

5. Zusammensetzung zum Verwenden derselben nach einem der vorhergehenden Ansprüche 1 bis 4, wobei die Zusammensetzung in flüssiger Form zum Sprühen oder zum Verwenden in einem Becken vorliegt.

6. Zusammensetzung zum Verwenden derselben nach einem der vorhergehenden Ansprüche 1 bis 5, wobei die Zusammensetzung für 50 bis 60 Tiere in mindestens 1 Liter bis höchstens 5 Liter Flüssigkeit verdünnt wird.

7. Zusammensetzung zum Verwenden derselben nach einem der vorhergehenden Ansprüche 1 bis 6, wobei die Zusammensetzung auf den Fuß von Huftieren einmal alle 3 Tage bis einmal alle 15 Tage aufgetragen wird.

8. Zusammensetzung nach einem der vorhergehenden Ansprüche 1 bis 7 in Verbindung mit mindestens einem Desinfektionsmittel zum Verwenden bei der Behandlung von Pathologien, die eine parasitäre oder bakterielle Infektion des Fußes von Huftieren beinhalten, wobei das mindestens eine Desinfektionsmittel mindestens 48 Stunden vor der Verwendung der Zusammensetzung verwendet wird.

## Claims

1. A composition comprising a bacterial mixture for use in the prevention and improvement of the condition of ungulates suffering from diseases involving a parasitic or bacterial podal infection, said composition comprising:
- at least one of the four following *Bacillus subtilis* strains: NOL01, NOL02, NOL03 and NOL04, said strains being deposited at the CNCM under the numbers CNCM I - 4606, CNCM I-5043, CNCM I - 4607 and CNCM I - 4608, respectively, and
- at least one lactic bacterium selected from the following lactic bacteria: *Lactococcus lactis spp lactis 1* strain NOL11 and *Pediococcus pentosaceus* 2 strain NOL12, said strains being deposited at the CNCM under the numbers CNCM I - 4609 and CNCM I - 4610, respectively.

2. The composition for use according to claim 1, said composition containing from 10⁵ to 10¹¹ bacterial *Bacillus* colonies and from 10⁵ to 10¹¹ lactic-bacteria bacterial colonies, the bacterial colonies being by ml or g of composition.

3. The composition for use according to claim 1 or claim 2, wherein said at least one Bacillus strain is in sporulated form and/or in vegetative form.

4. The composition for use according to any of claims 1 to 3, wherein said diseases involving a parasitic or bacterial infection in the feet of ungulates are selected from Mortellaro disease, interdigital dermatitis, or tyloma, paronychia or laminitis, or foot rot, bulb erosion, toe necrosis and pododermatitis.

5. The composition for use according to any of claims 1 to 4, said composition being in a liquid form that is intended to be sprayed or used in a basin.

6. The composition for use according to any of claims 1 to 5, wherein said composition is diluted in at least one 1 liter of liquid to at most 5 liters of liquid for 50 to 60 animals.

7. The composition for use according to any of claims 1 to 6, wherein said composition is applied to the feet of the ungulates from once every 3 days to once every 15 days.

8. The composition according to any of claims 1 to 7, in association with at least one disinfectant, for use in the context of treating diseases involving a parasitic or bacterial podal infection of the ungulates,
said at least one disinfectant being used at least 48 hours before said composition is used.
